**Europäisches Patentamt**

**European Patent Office**

**Office européen des brevets**

(19)

(11) Veröffentlichungsnummer : **0 364 842 B1**

(12) # EUROPÄISCHE PATENTSCHRIFT

(45) Veröffentlichungstag der Patentschrift :
07.10.92 Patentblatt 92/41

(51) Int. Cl.⁵ : **A61L 2/18**

(21) Anmeldenummer : **89118675.1**

(22) Anmeldetag : **07.10.89**

(54) **Verfahren zur Sterilisierung von teilchenförmigen pharmazeutischen Substanzen.**

(30) Priorität : **10.10.88 DE 3834371**

(43) Veröffentlichungstag der Anmeldung :
**25.04.90 Patentblatt 90/17**

(45) Bekanntmachung des Hinweises auf die
Patenterteilung :
**07.10.92 Patentblatt 92/41**

(84) Benannte Vertragsstaaten :
**AT BE CH DE ES FR GB IT LI NL SE**

(56) Entgegenhaltungen :
**DE-A- 2 638 355**

(73) Patentinhaber : **Gustmann-Westphalen,
Hartmut
Jahnstrasse 6
W-2300 Kiel 1 (DE)**

(72) Erfinder : **Gustmann-Westphalen, Hartmut
Jahnstrasse 6
W-2300 Kiel 1 (DE)**

(74) Vertreter : **UEXKÜLL & STOLBERG
Patentanwälte
Beselerstrasse 4
W-2000 Hamburg 52 (DE)**

**Beschreibung**

Die Erfindung betrifft ein Verfahren zur Sterilisierung von teilchenförmigen pharmazeutischen Substanzen, insbesondere von thermisch instabilen pharmazeutischen Substanzen wie Makrolid- und Polyen-Makrolid-Antibiotika mit Kaltsterilisatoren.

Aufgrund der Probleme bei der Sterilisierung von pharmazeutischen Substanzen mit Ethylenoxid, die in einigen Ländern zum Verbot der Verwendung von Ethylenoxid geführt haben, sind bei bestimmten pharmazeutischen Substanzen Lieferengpässe aufgetreten, da noch kein geeignetes Ersatzverfahren zur Sterilisierung gefunden worden ist. Dies gilt insbesondere für die Sterilisierung von thermisch instabilen pharmazeutischen Substanzen wie den Makroliden, insbesondere Polyen-Makroliden, wie z.B. Nystatin, Amphotericin B und ähnlichen.

Als Ersatz für Ethylenoxid bietet sich der Einsatz von gasförmigen Dialkyldicarbonaten (vgl. z.B. DE-OS 26 38 355) an. Diese aus dem Stand der Technik bekannte Vorgehensweise bringt jedoch verschiedene Nachteile mit sich und führt nicht immer zu befriedigenden Ergebnissen. So beobachtet man, daß bei Verwendung von gasförmigen Dialkyldicarbonaten zur Sterilisierung von teilchenförmigen pharmazeutischen Substanzen an der Oberfläche der Feststoffteilchen eine Kondensation der Dialkyldicarbonate eintritt, während tiefer liegende Teile der Substanz unsteril bleiben. Darüber hinaus besitzen flüssige Dialkyldicarbonate gegenüber bestimmten funktionellen Gruppen eine sehr große Reaktivität, so daß wegen der geschilderten Kondensation gasförmige Dialkyldicarbonate für die Sterilisierung von pharmazeutischen Substanzen in der Praxis bisher nicht eingesetzt worden sind. Schließlich ist auch der Einsatz von gasförmigen Dialkyldicarbonaten apparativ verhältnismäßig aufwendig.

Der Erfindung liegt deshalb die Aufgabe zugrunde, ein Verfahren zur Sterilisierung von teilchenförmigen pharmazeutischen Substanzen vorzuschlagen, das einfacher durchzuführen ist und bessere Ergebnisse liefert als die Sterilisierung mit gasförmigen Dialkyldicarbonaten.

Zur Lösung wird ein Verfahren zur Sterilisierung von teilchenförmigen pharmazeutischen Substanzen, insbesondere Makrolid- und Polyen-Makrolid-Antibiotika mit Kaltsterilisatoren vorgeschlagen, das dadurch gekennzeichnet ist, daß man die zu sterilisierende Substanz in einem inerten organischen Lösungsmittel suspendiert, aufschlämmt oder löst, dem Lösungsmittel vor oder nach der Einbringung der zu sterilisierenden Substanz Dialkyldicarbonat, insbesondere Dimethylcarbonat, oder $\beta$-Propiolacton als Kaltsterilisator zusetzt, den Kaltsterilisator für eine angemessene Sterilisierung ausreichend lange auf die zu sterilisierende Substanz einwirken läßt und dann den Kaltsterilisator und das Lösungsmittel entfernt.

Die Entfernung des Kaltsterilisators erfolgt entweder mit dem Abfiltrieren des Lösungsmittels und durch Nachwaschen. Als gut geeignet hat sich aber auch die Entfernung durch Hydrolyse oder Derivatisierung des Dialkyldicarbonats, z.B. mit Alkohol, erwiesen.

Als Kaltsterilisatoren können erfindungsgemäß Dialkyldicarbonate wie beispielsweise Dimethyldicarbonat und Diethyldicarbonat oder $\beta$-Propiolacton verwendet werden.

Inerte organische Lösungsmittel im Sinne der vorliegenden Erfindung sind alle Lösungsmittel, in denen sich die zu sterilisierende Substanz nicht oder nur schwer löst oder in denen sich die zu sterilisierende Substanz löst, das Lösungsmittel aber eine zu erwartende Reaktion zwischen der zu sterilisierenden Substanz und dem Kaltsterilisator sehr stark verlangsamt oder unterbindet. Geeignete inerte organische Lösungsmittel sind zum Beispiel Kohlenwasserstoffe, Ether, Ketone, Ester usw. sowie Mischungen dieser Lösungsmittel, soweit sie nicht oder nur langsam mit dem Kaltsterilisator reagieren. Vorzugsweise werden Lösungsmittel mit niedrigem Siedepunkt oder solche Lösungsmittel eingesetzt, die sich durch leicht flüchtige Lösungsmittel wie Kohlenwasserstoffe oder Ether entfernen lassen. Die Wahl des Lösungsmittels wird auch unter dem Gesichtspunkt getroffen, daß sich die zu sterilisierende Substanz in diesem Lösungsmittel nicht oder nur sehr schwer löst, so daß vorzugsweise eine Aufschlämmung und keine Lösung erhalten wird. Durch eine derartige Wahl des Lösungsmittels ist beispielsweise gewährleistet, daß eine durch Vermahlen vorgegebene Korngröße der zu sterilisierenden Substanz durch die Sterilisierung nicht verändert wird. Als besonders geeignet haben sich Kohlenwasserstoffe wie Pentan erwiesen.

Der Kaltsterilisator wird dem Lösungsmittel vor oder nach der Einbringung der zu sterilisierenden pharmazeutischen Substanz zugesetzt. Nach definierter Sterilisierungsdauer wird der Kaltsterilisator entfernt oder zerstört. Die Sterilisierungsdauer richtet sich nach dem Grad und der Art der Verkeimung, der Temperatur sowie der Konzentration des eingesetzten Kaltsterilisators und beträgt bei Raumtemperatur etwa 4 bis 6 Stunden. Bei geringen Konzentrationen des Kaltsterilisators kann die erforderliche Sterilisationsdauer bis zu 12 Stunden betragen. Das Auffinden der für den jeweiligen Fall besten Sterilisierungsdauer ist für den Fachmann unproblematisch und kann mit Hilfe weniger Vorversuche erfolgen.

Die Entfernung des Kaltsterilisators erfolgt im einfachsten Fall durch Filtration der suspendierten Substanz und Nachwaschen mit einem vorzugsweise leicht flüchtigen Lösungsmittel. Die Zerstörung des Kaltsterilisa-

2

tors erfolgt durch Hydrolyse oder Derivatisierung. Als Derivatisierungsmittel eignen sich zum Beispiel Alkohole. Das Lösungsmittel wird durch Filtration, Zentrifugation oder Destillation (Vakuumdestillation) entfernt.

Der Temperaturbereich, in dem die Sterilisierung durchgeführt werden kann, hängt selbstverständlich von der Temperaturempfindlichkeit der zu sterilisierenden Substanz ab. Davon abgesehen ist der Temperaturbereich ausschließlich von der Wahl des Lösungsmittels abhängig. Nach oben hin ist der Temperaturbereich durch den Siedepunkt des eingesetzten Lösungsmittels festgelegt, während der Temperaturbereich nach unten hin durch die Temperatur begrenzt ist, bei der das eingesetzte Dialkyldicarbonat in dem Lösungsmittel noch in hinreichender Konzentration gelöst und reaktiv ist. Dabei kann der untere Temperaturbereich zum Beispiel für Dimethyldicarbonat durch die Anwendung eines Lösungsmittels wie Pentan beträchtlich unter dem Erstarrungspunkt des Dimethyldicarbonats liegen. Normalerweise wird die Sterlisierung jedoch bei Raumtemperatur durchgeführt.

Gegenüber der Verwendung von gasförmigen Dialkyldicarbonaten hat das erfindungsgemäße Verfahren erhebliche Vorteile. Zum einen ist der apparative Aufwand beim Arbeiten mit Lösungen des Sterilisierungsmittels geringer als bei Verwendung von gasförmigem Sterilisierungsmittel. Darüber hinaus kann der Kaltsterilisator beim erfindungsgemäßen Verfahren homogen in jeder gewünschten Konzentration exakt dosiert werden. Dies bietet gegenüber der Verwendung von gasförmigen Dialkyldicarbonaten den Vorteil, daß ein gleichmäßiges Einwirken des Sterilisierungsmittels gegenüber der gesamten Substanzmenge gewährleistet ist. Darüber hinaus besteht beim erfindungsgemäßen Verfahren nicht die Gefahr, daß örtlich sehr hohe Dialkyldicarbonatkonzentrationen (Kondensation der gasförmigen Dialkyldicarbonate an der Substanzoberfläche) auftreten, so daß chemische Reaktionen mit der zu sterilisierenden Substanz vernachlässigbar sind, da durch die Verdünnung mit dem organischen Lösungsmittel die Reaktivität des Kaltsterilisators sehr stark vermindert ist. Schließlich hat sich gezeigt, daß bei der Sterilisierung von Antibiotika, z.B. Nystatin, die Abnahme der Aktivität des Antibiotikums geringer als bei der Sterilisierung mit Ethylenoxid ist. Bei der Sterilisierung mit Ethylenoxid ist z.B. bei Nystatin eine Aktivitätsabnahme von etwa 8 bis 10% zu beobachten. Bei der erfindungsgemäßen Sterilisierung mit Dimethyldicarbonat tritt nur eine Aktivitätsabnahme von etwa 5% auf.

Wie sich bereits aus den obigen Ausführungen ergibt, ist das erfindungsgemäße Verfahren besonders für die Sterilisierung von thermisch instabilen pharmazeutischen Substanzen geeignet. Bevorzugt wird das erfindungsgemäße Verfahren dem- entsprechend auf Makrolid- und Polyen-Makrolid-Antibiotika angewendet, bei denen die eingangs geschilderten Probleme zur Zeit am größten sind. Entsprechend einer bevorzugten Ausführungsform des erfindungsgemäßen Verfahrens wird dementsprechend Nystatin oder Amphotericin B sterilisiert.

## Beispiel 1

20 g verkeimtes Nystatin wurden in 200 ml Pentan, dem 1 Gew.% Dimethyldicarbonat zugesetzt worden waren, aufgeschlämmt. Es wurde 6 Stunden lang gerührt. Danach wurde das suspendierte Nystatin abgesaugt, mit 180 ml sterilfiltriertem Pentan nachgewaschen und trockengesaugt. Die Sterilität des Nystatins wurde nach einer Einwirkungsdauer von 6 Stunden durch Sterilkontrolle nachgewiesen.

## Beispiel 2

20 g verkeimtes Nystatin wurden in 180 ml Pentan aufgeschlämmt. Zu dieser Aufschlämmung wurden 20 ml einer Lösung gegeben, die aus Pentan und 1 Gew.% Dimethyldicarbonat bestand. Es wurde 12 Stunden gerührt. Dann wurde das suspendierte Nystatin abgesaugt, mit 180 ml sterilfiltriertem Pentan nachgewaschen und trockengesaugt. Die Sterilität des Nystatins wurde durch Sterilkontrolle nachgewiesen.

## Beispiel 3

Beispiel 1 wurde wiederholt mit dem Unterschied, daß nach der 6-stündigen Sterilisierungsdauer 2 ml Ethanol zugesetzt wurden und anschließend 24 Stunden lang gerührt wurde. Dadurch wurde alles Dimethyldicarbonat derivatisiert. Das suspendierte Nystatin wurde abgesaugt, mit 50 ml Pentan nachgewaschen und getrocknet. Das so erhaltene Nystatin war steril.

## Beispiel 4

Beispiel 2 wurde wiederholt mit dem Unterschied, daß nach Ablauf der 12-stündigen Sterilisierungsdauer 2 ml Ethanol zugefügt wurden und 24 Stunden lang gerührt wurde. Dadurch wurde alles Dimethyldicarbonat derivatisiert. Das suspendierte Nystatin wurde dann abgesaugt, mit 50 ml Pentan nachgewaschen und getrocknet. Auch das so behandelte Nystatin war steril.

**Patentansprüche**

1. Verfahren zur Sterilisierung von teilchenförmigen pharmazeutischen Substanzen, insbesondere Makrolid- und Polyen-Makrolid-Antibiotika, mit Kaltsterilisatoren, **dadurch gekennzeichnet**, daß man die zu sterilisierende Substanz in einem inerten organischen Lösungsmittel suspendiert, aufschlämmt oder löst, dem Lösungsmittel vor oder nach der Einbringung der zu sterilisierenden Substanz Dialkylcarbonat, insbesondere Dimethylcarbonat, oder β-Propiolacton als Kaltsterilisator zusetzt, den Kaltsterilisator für eine angemessene Sterilisierung ausreichend lange auf die zu sterilisierende Substanz einwirken läßt und dann den Kaltsterilisator und das Lösungsmittel entfernt.

2. Verfahren nach Anspruch 1, **dadurch gekennzeichnet,** daß man Nystatin oder Amphotericin B sterilisiert.

**Claims**

1. Procedure for sterilizing pharmaceutical substances in particle form, particularly macrolide and polymacrolide antibiotics, using cold sterilizing agents, characterized in that the substance to be sterilized is suspended, reduced to a paste or dissolved in an inert organic solvent, dialkyl carbonate, particularly dimethyl carbonate or β-propiolacton is added to the solvent as cold sterilizing agent before or after introduction of the substance to be sterilized, the cold sterilizing agent is left to act on the substance to be sterilized for a suitable length of time to ensure appropriate sterilization and then the cold sterilizing agent and the solvent are removed.

2. Procedure in accordance with Claim 1, characterized in that Nystatin or Amphotericin B are sterilized.

**Revendications**

1. Procédé pour stériliser des substances pharmaceutiques particulaires, en particulier des antibiotiques de types macrolides et macrolides polyènes, avec des stérilisateurs à froid, caractérisé en ce qu'on met la substance à stériliser en suspension dans un solvant organique inerte, on la disperse ou on la dissout, on ajoute au solvant, avant ou après introduction de la substance à stériliser, un dialkylcarbonate, en particulier un diméthylcarbonate, ou de la β-propiolactone, comme stérilisateur à froid, on fait agit le stérilisateur à froid sur la substance à stériliser suffisammment longtemps pour obtenir une stérilisation convenable, et on élimine ensuite le stérilisateur à froid et le solvant.

2. Procédé selon la revendication 1, caractérisé en ce qu'on stérilise de la nystatine ou de l'amphotéricine B.